# EUROPEAN PATENT APPLICATION

(11) **EP 4 410 202 A1**
(43) Date of publication of application: **07.08.2024**
(21) Application number: 22876147.4
(22) Date of filing: 26.09.2022
(51) Int. Cl.: A61B 5/16, A61B 5/00, G06Q 50/10, G16H 20/70, G16H 80/00

(54) **BIOMETRIC INFORMATION PROCESSING PROGRAM, BIOMETRIC INFORMATION PROCESSING DEVICE, BIOMETRIC INFORMATION PROCESSING SYSTEM, AND BIOMETRIC INFORMATION PROCESSING METHOD**

(30) Priority: 28.09.2021 JP 2021157513; 12.04.2022 JP 2022065511
(71) Applicant: Nitto Denko Corporation, Ibaraki-shi, Osaka 567-8680 (JP)
(72) Inventor: MINAKATA, Masayuki, Ibaraki-shi, Osaka 567-8680 (JP); OKADA, Kazumasa, Ibaraki-shi, Osaka 567-8680 (JP); KOZONOI, Nobuyuki, Ibaraki-shi, Osaka 567-8680 (JP); YAMAMOTO, Masayasu, Ibaraki-shi, Osaka 567-8680 (JP)
(74) Representative: Hoffmann Eitle
(86) International application number: PCT/JP2022/035773
(87) International publication number: WO 2023/054280

(57) **Abstract**

A biological information processing program causes a computer to execute a process including obtaining biological information of a user from a biological information obtainment device, and causing a display device to display both a waveform indicating time-series change in the biological information and information indicating a result of processing using a predetermined number of pieces of the biological information.

## Description

### TECHNICAL FIELD

The present invention relates to biological information processing programs, biological information processing devices, biological information processing systems, and biological information processing methods.

### BACKGROUND ART

A system configured to generate stress data indicating time-series change in a stress level of a user from biological information and behavioral history of the user and to estimate whether or not the user has interpersonal stress caused by the user's contact with the others, is known.

In addition, counseling in which a counselor and an individual talk about psychological issues, such as troubles, anxieties, and the like of the individual, and the counselor provides the individual with assistance and advice from a professional point of view based on his or her expertise, is known.

### CITATION LIST

### PATENT LITERATURE

Patent Literature 1: Japanese Patent Publication No. 2018-037073

### SUMMARY OF INVENTION

### TECHNICAL PROBLEM

In counseling, generally, a counselor reads an individual's psychological state based on his or her facial expression, gaze, tone of voice, gestures, and the like of an individual who is receiving counseling, and gives advice and the like to that individual in accordance with the psychological state. Therefore, quality of counseling depends on skills of the counselor, and the quality of counseling varies. In one aspect, it is an object to improve the quality of counseling.

### SOLUTION TO PROBLEM

One aspect is related to a biological information processing program that causes a computer to execute a process including: obtaining biological information of a user from a biological information obtainment device; and causing a display device to display both a waveform indicating time-series change in the biological information and information indicating a result of processing using a predetermined number of pieces of the biological information.

### ADVANTAGEOUS EFFECTS OF INVENTION

It is possible to improve the quality of counseling.

### BRIEF DESCRIPTION OF DRAWINGS

[FIG. 1] FIG. 1 is a diagram illustrating an example of a system configuration of a biological information processing system of a first embodiment.
[FIG. 2] FIG. 2 is a diagram describing fluctuation in a stress value during counseling.
[FIG. 3] FIG. 3 is a diagram describing an example of a hardware configuration of an information processing device.
[FIG. 4] FIG. 4 is a diagram describing functions of devices included in a biological information processing system.
[FIG. 5] FIG. 5 is a first flowchart describing a process performed by the information processing device.
[FIG. 6] FIG. 6 is a first diagram illustrating an example of display of the information processing device.
[FIG. 7] FIG. 7 is a second diagram illustrating an example of display of the information processing device.
[FIG. 8] FIG. 8 is a second flowchart describing a process performed by the information processing device.
[FIG. 9] FIG. 9 is a third diagram illustrating an example of display of the information processing device.
[FIG. 10] FIG. 10 is a diagram illustrating an example of a system configuration of a biological information processing system of a second embodiment.
[FIG. 11] FIG. 11 is a sequence diagram illustrating a process performed by the biological information processing system of the second embodiment.
[FIG. 12] FIG. 12 is a diagram illustrating an example of a counselor's screen.

### DESCRIPTION OF EMBODIMENTS

### (First Embodiment)

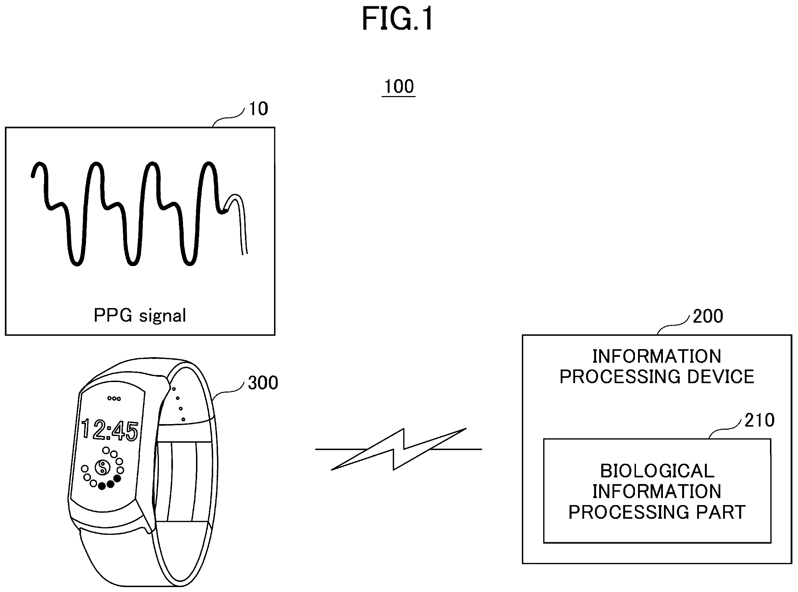

Embodiments will be described below with reference to the drawings. FIG. 1 is a diagram illustrating an example of a system configuration of a biological information processing system of the first embodiment.

A biological information processing system 100 of the present embodiment includes an information processing device 200 and a wearable terminal 300. The information processing device 200 and the wearable terminal 300 communicate wirelessly or the like.

The biological information processing system 100 of the present embodiment is used, for example, at a physical location where the counseling takes place. Specifically, in the biological information processing system 100, for example, the information processing device 200 may be a tablet-type terminal, a smartphone, or the like that is mainly used by a counselor who performs counseling.

The wearable terminal 300 of the present embodiment is attached to a counseling subject (individual) who receives counseling, and detects biological information 10 of the counseling subject during counseling. The wearable terminal 300 transmits the detected biological information 10 to the information processing device 200. In other words, the wearable terminal 300 is an example of a biological information obtainment device configured to obtain biological information of a counseling subject wearing the wearable terminal 300.

The biological information 10 of the present embodiment may be, for example, a pulse wave or the like of a counseling subject. In the example of FIG. 1, the wearable terminal 300 is a wristwatch-type terminal, but is not limited to this. The wearable terminal 300 may be in any form as long as the biological information of the counseling subject can be obtained. The counseling subject of the present embodiment is an example of a user of the wearable terminal 300 (biological information obtainment device) .

The biological information 10 of the present embodiment may be obtained from, for example, image data of the counseling subject. In this case, the biological information obtainment device may be a terminal device or the like including a capturing device. The terminal device may be a portable terminal device, such as a tablet, a smartphone, or the like. The terminal device may also be a desktop personal computer, and may be in any form as long as the terminal device includes a capturing device.

The biological information 10 obtained from the image data of the counseling subject includes, for example, heart rate, heart rate variability, stress level, blood oxygen concentration, respiratory rate, blood pressure, and the like of the counseling subject.

The information processing device 200 of the present embodiment includes a biological information processing part 210. When the biological information 10 is obtained from the wearable terminal 300, a stress value indicating the stress of the counseling subject as a numerical value based on the biological information 10, is calculated. The stress value is a value calculated using the biological information and is an index value indicating the magnitude of a psychological stress of the user of the wearable terminal 300.

Then, the information processing device 200 causes a display to display: a waveform indicating time-series fluctuation in the stress value of the counseling subject during counseling; and information indicating a trend of fluctuation in the stress value of the counseling subject during counseling.

In the present embodiment, by displaying the change in the stress value of the counseling subject during counseling in real time, the counselor can understand a psychological state of the counseling subject.

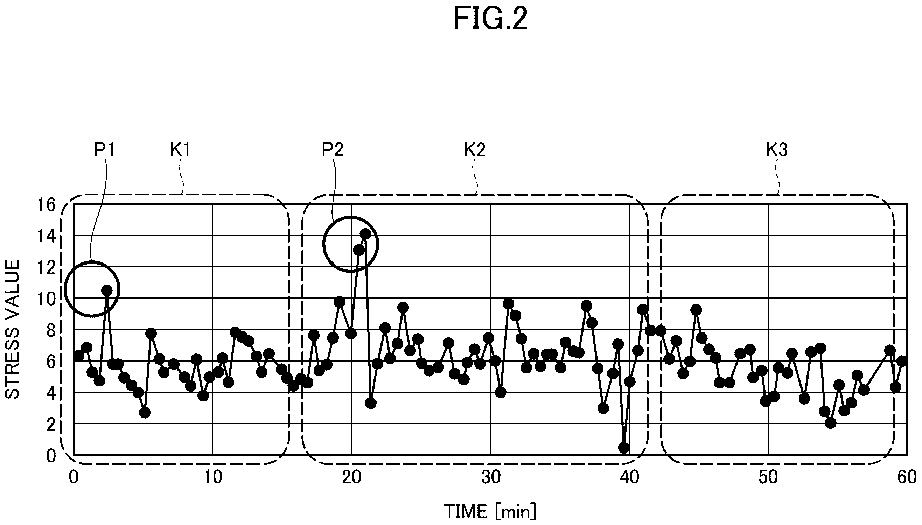

The fluctuation in the stress value of the counseling subject during counseling will be described below with reference to FIG. 2. FIG. 2 is a diagram describing fluctuation in the stress value during counseling.

During a predetermined period of time, counseling is conducted, for example, through a dialogue to build a relationship between the counseling subject and the counselor, a dialogue related to the core of an issue (questioning), a dialogue to ease nervousness (mindfulness), and the like.

In FIG. 2, the horizontal axis indicates time and the vertical axis indicates the stress value. FIG. 2 indicates fluctuation in the stress value of the counseling subject during counseling.

In the waveform as illustrated in FIG. 2, a section K1 indicates fluctuation in the stress value of the counseling subject during a time range in which the dialogue to build the relationship between the counseling subject and the counselor is conducted. A section K2 indicates fluctuation in the stress value of the counseling subject during a time range in which the dialogue related to the core of an issue is conducted. A section K3 indicates fluctuation in the stress value of the counseling subject during a time range in which the dialogue to reduce tension is conducted.

In the section K1, the stress value reaches the maximum value P1 shortly after the start of counseling, and the stress value gradually decreases from the maximum value P1. Therefore, judging from this trend of fluctuation, it can be inferred that the relationship with the counselor is being built steadily, and the counseling subject is relaxing.

In the section K2, the stress value gradually increases and reaches the maximum value P2 at a certain timing. The maximum value P2 is larger than the maximum value P1 in the section K1. Judging from this trend of fluctuation, it can be inferred that the dialogue is related to the core of the issue, and the tension of the counseling subject has increased.

In the section K3, the stress value gradually decreases. Judging from this trend of fluctuation, it can be inferred that the tension that increased in the section K2 is being relieved.

In the present embodiment, the trend of fluctuation in the stress value as illustrated in FIG. 2 is shown to the counselor during counseling. In other words, in the present embodiment, the trend of fluctuation in the stress value reflecting the psychological state of the counseling subject is shown to the counselor.

Thus, in the present embodiment, the counselor can infer the psychological state of the counseling subject, and can readily determine whether or not the currently conducted dialogue is suitable for the psychological state of the counseling subject.

In addition, the information processing device 200 of the present embodiment displays, on the display, information indicating the trend of the fluctuation in the stress value together with the waveform as illustrated in FIG. 2.

Therefore, according to the present embodiment, the counselor can visually understand the trend of the fluctuation in the stress value in a short time, and does not inhibit the counselor from observing behaviors of the counseling subject.

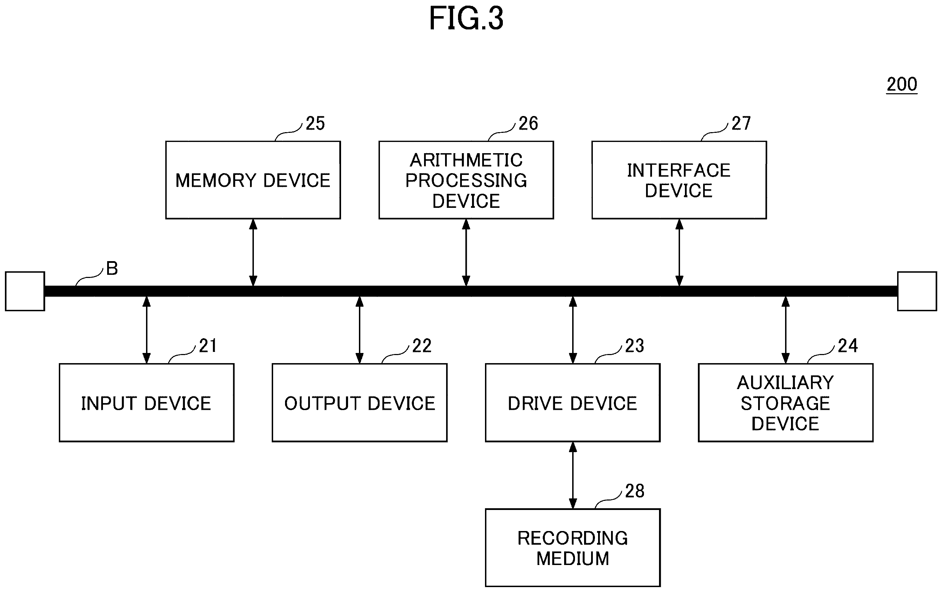

The information processing device 200 of the present embodiment will be described below. FIG. 3 is a diagram describing an example of a hardware configuration of the information processing device.

The information processing device 200 of the present embodiment is a computer including an input device 21, an output device 22, a drive device 23, an auxiliary storage device 24, a memory device 25, an arithmetic processing device 26, and an interface device 27, which are mutually connected by a bus B.

The input device 21 is a device configured to input various types of information, and is realized by a touch panel or the like. The output device 22 is configured to output various types of information, and is realized by a display (display device) or the like. The interface device 27 includes a LAN card or the like, and is used for connection to a network.

A biological information processing program that realizes the biological information processing part 210 of the information processing device 200 is at least a part of various programs that control the information processing device 200. The biological information processing program is provided by, for example, distributing a recording medium 28 or downloading the biological information processing program from a network. The recording medium 28 in which the biological information processing program is recorded can be various types of recording media that record information optically, electrically, or magnetically, such as a CD-ROM, a flexible disk, a magneto-optical disk, or the like; or a semiconductor memory that records information electrically, such as a ROM, a flash memory, or the like.

When the recording medium 28 in which the biological information processing program is recorded is set in the drive device 23, the biological information processing program recorded in the recording medium 28 is installed from the recording medium 28 to the auxiliary storage device 24 via the drive device 23. A health condition management program downloaded from the network is installed to the auxiliary storage device 24 via the interface device 27.

The auxiliary storage device 24 included in the information processing device 200 stores the biological information processing program installed in the information processing device 200 also stores various files, data, and the like necessary for the information processing device 200. Upon start-up of the information processing device 200, the memory device 25 reads, and stores, the biological information processing program from the auxiliary storage device 24. The arithmetic processing device 26 realizes various processes as described below in accordance with the biological information processing program stored in the memory device 25.

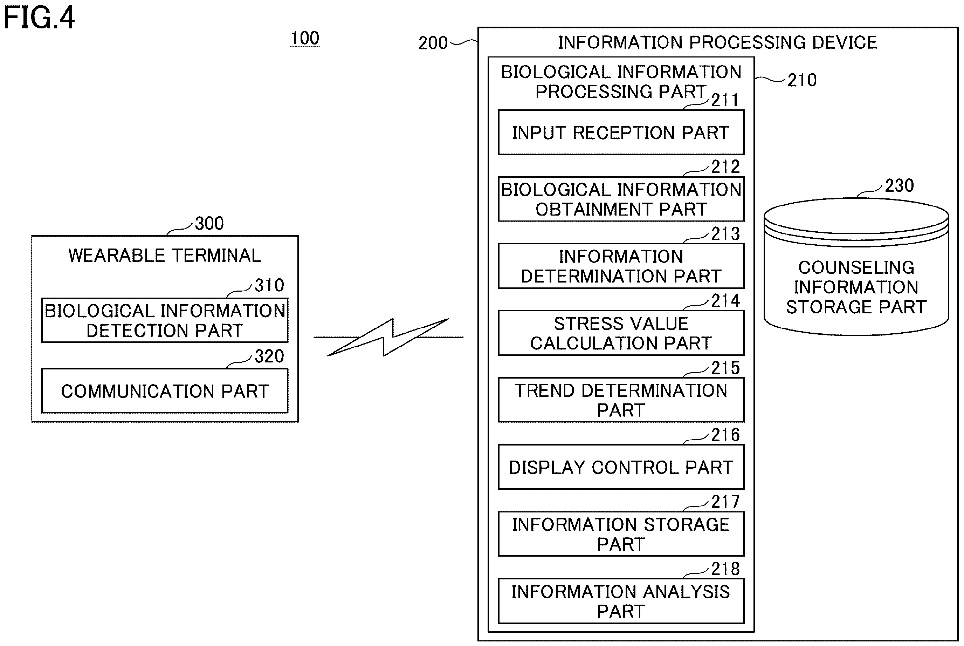

Next, the functions of the devices of the biological information processing system 100 of the present embodiment will be described with reference to FIG. 4. FIG. 4 is a diagram describing the functions of the devices included in the biological information processing system.

First, the functions of the information processing device 200 of the present embodiment will be described. The information processing device 200 of the present embodiment includes the biological information processing part 210 and a counseling information storage part 230.

The biological information processing part 210 includes an input reception part 211, a biological information obtainment part 212, an information determination part 213, a stress value calculation part 214, a trend determination part 215, a display control part 216, an information storage part 217, and an information analysis part 218.

The input reception part 211 is configured to receive various inputs to the information processing device 200. Specifically, the input reception part 211 receives inputs in accordance with operations by the counselor.

The biological information obtainment part 212 is configured to obtain biological information transmitted from the wearable terminal 300. The information determination part 213 is configured to determine whether or not the biological information obtained by the biological information obtainment part 212 is information that can be used for calculation of the stress value.

The stress value calculation part 214 is configured to calculate a stress value of the counseling subject wearing the wearable terminal 300, using the biological information.

Specifically, the stress value calculation part 214 calculates a stress value using, for example: an index value derived from a peak value of a pulse wave and a time between peaks obtained as the biological information of the counseling subject; and a pulse pressure correlated with the index value.

The trend determination part 215 is configured to determine a trend of the fluctuation in the stress value using a predetermined number of stress values obtained by the biological information obtainment part 212. In other words, the trend determination part 215 determines a trend of change in the biological information using a predetermined number of pieces of the biological information. For example, the predetermined number of stress values may be five stress values or may be stress values obtained for five minutes.

The display control part 216 is configured to control display of the information processing device 200. In other words, the display control part 216 causes the display device (display) to display: the waveform indicating change in the biological information; and the information indicating the result of the processing performed by the trend determination part 215 using the predetermined number of the pieces of the biological information.

The information storage part 217 is configured to store, as counseling information, the biological information obtained by the biological information obtainment part 212, the stress value calculated by the stress value calculation part 214, the determination result obtained by the trend determination part 215, the information received by the input reception part 211, and the like, in the counseling information storage part 230. The information analysis part 218 is configured to analyze the counseling information stored in the counseling information storage part 230.

The counseling information storage part 230 of the present embodiment is configured to store the counseling information. The counseling information of the present embodiment indicates information obtained during a period from the start until the end of counseling of the counseling subject.

Specifically, the counseling information includes, for example, biological information, stress values, trend of fluctuation in the stress value, analysis results of the stress values, text data input by the counselor, and the like. The counseling information may also include identification information for identifying the counseling subject and information for identifying the date and time of counseling.

Next, functions of the wearable terminal 300 will be described. The wearable terminal 300 is a computer including an arithmetic processing device and a memory device.

The wearable terminal 300 includes a biological information detection part 310 and a communication part 320. The biological information detection part 310 is configured to detect biological information of a counseling subject wearing the wearable terminal 300. Specifically, the biological information detection part 310 may detect change in the volume of blood vessels of the counseling subject as the biological information by a photoelectric volumetric pulse wave recording method or the like.

Further, the wearable terminal 300 of the present embodiment includes an acceleration sensor. An output value of the acceleration sensor may be included in the biological information as information indicating movements of the counseling subject wearing the wearable terminal 300.

The communication part 320 is configured to communicate with the wearable terminal 300 and the information processing device 200. Specifically, the communication part 320 transmits the biological information detected by the biological information detection part 310, to the information processing device 200.

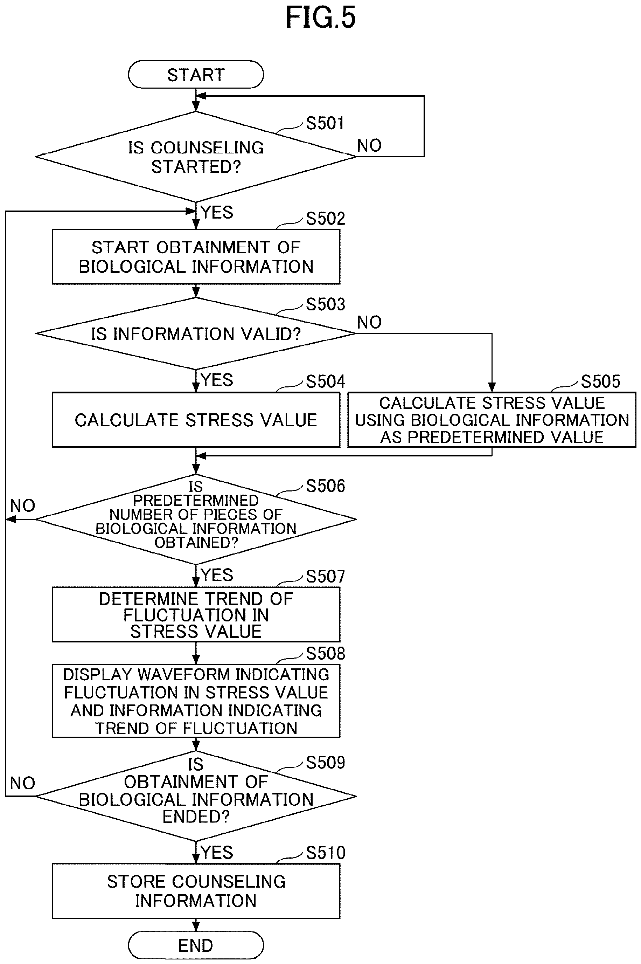

Next, a process performed by the information processing device 200 of the present embodiment will be described. First, the process performed by the information processing device 200 during counseling will be described with reference to FIG. 5. FIG. 5 is a first flowchart describing the process performed by the information processing device.

The information processing device 200 of the present embodiment determines whether or not counseling is started (step S501). Specifically, the information processing device 200 may determine that counseling is started, for example, when the input reception part 211 receives an operation or a signal indicating the start of counseling.

In step S501, when counseling is not started, the information processing device 200 stands by. In step S501, when counseling is started, the biological information obtainment part 212 of the information processing device 200 starts obtainment of biological information transmitted from the wearable terminal 300 (step S502).

Subsequently, the information determination part 213 of the information processing device 200 determines whether or not the obtained biological information is valid biological information that can be used for calculation of a stress value (step S503) .

Hereinafter, valid biological information and invalid biological information in the present embodiment will be described.

For example, when a fluctuation range of the output value of the acceleration sensor obtained from the wearable terminal 300 is equal to or greater than a predetermined threshold, the information determination part 213 of the present embodiment may determine that the obtained biological information is invalid information.

For example, when the counseling subject moves his or her body greatly by, for example, changing his or her posture, the fluctuation range of the output value of the acceleration sensor becomes equal to or greater than the predetermined threshold. In the present embodiment, the biological information obtained in such a state is not regarded as information reflecting the psychological state of the counseling subject, but is regarded as invalid information.

The information determination part 213 of the present embodiment may determine that the biological information is invalid information, for example, when the intensity of the signal obtained as the biological information is less than a predetermined lower limit or is equal to or greater than a predetermined upper limit.

The case in which the intensity of the signal obtained as the biological information is less than the predetermined lower limit indicates that no pulse wave is detected. The case in which the intensity of the signal obtained as the biological information is equal to or greater than the predetermined upper limit is, for example, considered to occur due to external disturbance or the like. Therefore, in the present embodiment, the above biological information is regarded as invalid information.

When the biological information is determined to be valid information in step S502, the stress value calculation part 214 of the information processing device 200 calculates a stress value using the biological information (step S504) and causes the process to proceed to step S506 that will be described below.

When the biological information is determined to be invalid information in step S502, the stress value calculation part 214 of the information processing device 200 uses a stress value obtained based on a most-recently performed calculation as the current stress value (step S505) and causes the process to proceed to step S506 that will be described below.

The trend determination part 215 of the information processing device 200 determines whether or not a predetermined number of pieces of the biological information is obtained (step S506). Here, the information processing device 200 determines whether or not a predetermined number of pieces of the biological information necessary for the trend determination part 215 to determine a trend of fluctuation in the stress value, is obtained.

When the predetermined number of the pieces of the biological information is not obtained in step S506, the information processing device 200 causes the process to return to step S502.

When the predetermined number of the pieces of the biological information is obtained in the predetermined number in step S506, the trend determination part 215 of the information processing device 200 determines the trend of the fluctuation in the stress value (step S507).

Specifically, for example, the trend determination part 215 compares the average value of the past several stress values including the most-recently calculated stress value with the stress value determined in step S504 or step S505. When the determined stress value is greater than the average value, the stress value calculation part 214 determines that the trend of the fluctuation in the stress value is an increasing trend. When the determined stress value is less than the average value, the trend determination part 215 determines that the trend of the fluctuation in the stress value is in a decreasing trend. Further, when the average value coincides with the determined stress value, the trend determination part 215 determines that the stress value does not fluctuate.

Subsequently, the display control part 216 of the information processing device 200 causes a display to display a waveform indicating the fluctuation in the stress value and information indicating the trend of the fluctuation in the stress value determined in step S507 (step S508).

Subsequently, the information processing device 200 determines whether or not to end obtainment of the biological information (step S509). Specifically, the input reception part 211 of the information processing device 200 determines whether or not an operation or signal indicating the end of counseling is received.

When the obtainment of the biological information is not ended in step S509, the information processing device 200 causes the process to return to step S502.

When the obtainment of the biological information is ended in step S509, the information storage part 217 of the information processing device 200 stores the counseling information in the counseling information storage part 230 (step S510) and ends the obtainment of the biological information.

At this time, the information storage part 217 stores, as a part of the counseling information, information identifying the counseling subject and information indicating the date and time of the counseling in the counseling information storage part 230.

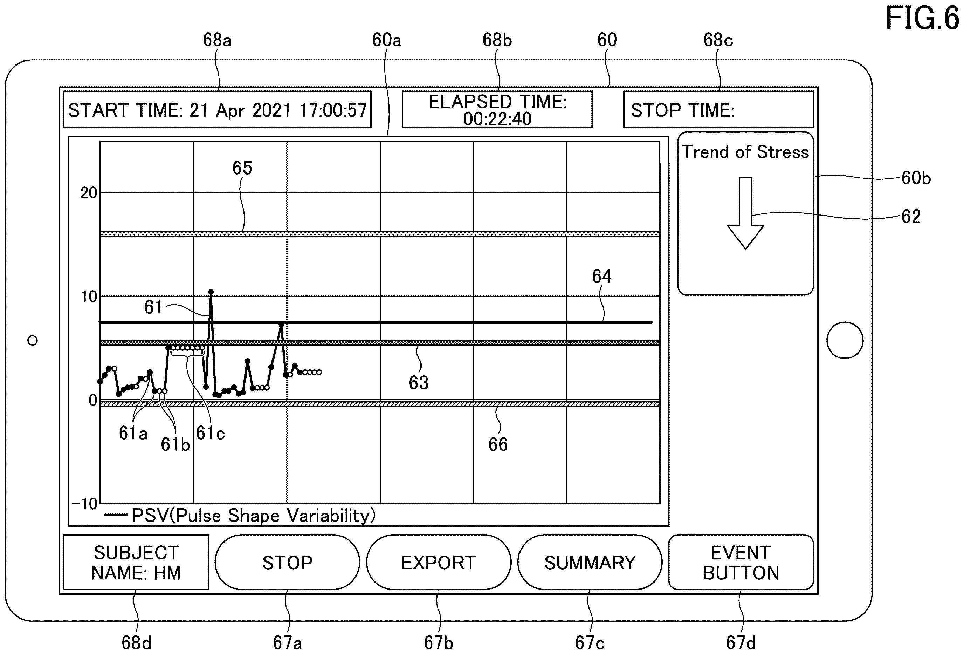

The above is the process performed by the information processing device 200 during counseling. An example of display of the information processing device 200 of the present embodiment will be described below with reference to FIG. 6. FIG. 6 is a first diagram illustrating an example of display of the information processing device. FIG. 6 illustrates an example of a screen displayed on the information processing device 200 in step S508 of FIG. 5. That is, the screen 60 of FIG. 6 illustrates an example of a screen displayed on the information processing device 200 during counseling.

The screen 60 includes display fields 60a and 60b, operation buttons 67a, 67b, 67c, and 67d, and display fields 68a, 68b, 68c, and 68d. The information processing device 200 includes an operation button 70 (input device).

In the display field 60a, a waveform 61 indicating the fluctuation in the stress value is displayed. The waveform 61 indicating the fluctuation in the stress value is, in other words, information indicating time-series change in the biological information of the user of the wearable terminal 300. In the display field 60b, an image 62 is displayed as information indicating the trend of the fluctuation in the stress value.

In the display field 60a of the present embodiment, a straight line 63 indicating the threshold of the stress value in the currently conducted counseling is displayed together with the waveform 61.

In the present embodiment, for example, when the stress value indicating the waveform 61 exceeds the threshold indicated by the straight line 63, it can be found that the tension of the counseling subject continues to be high.

In the display field 60a, a straight line 64 indicating the average value of the stress values in the most-recent prior counseling is displayed together with the waveform 61. Further, in the display field 60a, a straight line 65 indicating the maximum value of the stress values in the most-recent prior counseling and a straight line 66 indicating the minimum value of the stress values in the most-recent prior counseling are displayed.

In the present embodiment, by displaying the value indicating the fluctuation in the stress value in the most-recent prior counseling together with the waveform 61, the counselor can infer how the psychological state of the counseling subject changes from the psychological state in the most-recent prior counseling.

In the present embodiment, the threshold value of the stress value indicated by the straight line 63 is set to be smaller than the average value of the stress values in the most-recent prior counseling indicated by the straight line 64. When the stress value of the counseling subject during counseling is less than the threshold, it is inferred that the counseling subject is receiving counseling in a state that is more relaxing than in the most-recent prior counseling. In other words, in this case, it can be inferred that a good relationship is built between the counselor and the counseling subject in the most-recent prior counseling.

In the present embodiment, by setting the stress value during counseling in this manner, the counselor can infer whether or not a good relationship with the counseling subject is built in the most-recent prior counseling.

In the waveform 61 of the present embodiment, a display mode based on points indicating the stress values corresponding to the biological information determined to be valid is made different from a display mode of points indicating the stress values corresponding to the biological information determined to be invalid.

In the waveform 61 illustrated in FIG. 6, a point group 61a indicates the stress values calculated from the biological information determined to be valid, and point groups 61b and 61c indicate the stress values corresponding to the biological information determined to be invalid.

Each of the points included in the point group 61a is displayed with the point itself being filled, and each of the points included in the point groups 61b and 61c is displayed with blank points. In addition, in the present embodiment, when the biological information is determined to be invalid, the first stress value of the point group 61b is the same value as the stress value just before the first stress value. This is because the previously calculated stress value is used as the first stress value of the point group 61b. The same stress value is used until the obtained biological information is determined to be valid. The same applies to the point group 61c.

A method of determining the stress value when the biological information is determined to be invalid is not limited to the example described above. The stress value when the biological information is determined to be invalid may be, for example, an average value of a predetermined number of stress values calculated in the past. The method of determining the stress value when the biological information is determined to be invalid may be determined in a given manner.

As long as the display mode of the points indicating the stress values calculated from the biological information determined to be valid is different from the display mode of the points indicating the stress values corresponding to the biological information determined to be invalid, the display mode is not limited to the example as illustrated in FIG. 6.

In the present embodiment, the stress value is calculated from the biological information obtained from the wearable terminal 300, and the waveform of the stress value is displayed. However, this is by no means a limitation. In the present embodiment, the waveform indicating time-series change in the biological information may be displayed in the display field 60a. Specifically, for example, when the biological information is information indicting change in the volume of the blood vessel of the counseling subject, the time-series change in the volume of the blood vessel may be displayed in the form of a waveform.

In the display field 60b of the present embodiment, the image 62, which is information indicating the trend of fluctuation in the stress value, is displayed. In other words, the image 62 of the present embodiment is information indicating the trend of time-series change in the biological information, i.e., an image indicating an increase or decrease in the value calculated based on the biological information.

In the example of FIG. 6, the image 62 has an arrow shape. In the example of FIG. 6, because the image 62 of a downward arrow is displayed, it can be found that the fluctuation in the stress value is in a decreasing trend.

In the present embodiment, by displaying the trend of the fluctuation in the stress value with the image, the counselor can visually understand the trend of the fluctuation in the stress value in a short period of time without reducing the attention to the counseling subject.

The shape of the image 62 displayed in the display field 60b is not limited to the arrow illustrated in FIG. 6. The image 62 may be a given image as long as the image has a shape indicating the trend of the fluctuation in the stress value.

In the present embodiment, the image displayed in the display field 60b may be displayed in a different manner, for example, by blinking, color changing, or the like in response to change in the trend of the fluctuation in the stress value.

In the present embodiment, the image 62 is displayed on the screen 60 together with the waveform 61. However, this is by no means a limitation. During counseling, the information processing device 200 of the present embodiment may hide the waveform 61 and enlarge the image 62 by using the display field 60a, in which the waveform 61 is displayed, and the display field 60b.

By enlarging the image 62, the counselor can readily visually understand the trend of the fluctuation in the stress value.

In the present embodiment, the image 62 may be hidden and the waveform 61 alone may be displayed. Switching between a displayed state and a hidden state of the waveform 61 or the image 62 may be performed by the operation of the counselor.

In the present embodiment, for example, the switching between the displayed state and the hidden state of the waveform 61 or the image 62 may be performed by operating the operation button 70. The switching between the displayed state and the hidden state of the waveform 61 or the image 62 may be performed by a communication terminal or the like that is communicable with the information processing device 200. The communication terminal may be, for example, a transmitter or the like that can be placed near the counselor. In this case, by the counselor operating the communication terminal placed near the counselor, a signal for performing the switching between the displayed state and the hidden state of the waveform 61 or the image 62 may be transmitted from the communication terminal to the information processing device 200.

The operation button 67a is an operation button for stopping the obtainment of the biological information from the wearable terminal 300. The operation button 67a is operated, for example, when the counseling is ended. In a state before the start of the obtainment of the biological information, the operation button 67a is displayed as an operation button for starting the obtainment of the biological information from the wearable terminal 300.

The operation button 67b is an operation button for outputting the counseling information obtained during counseling. The output destination of the counseling information may be the counseling information storage part 230. The counseling information storage part 230 may be provided externally of the information processing device 200.

The operation button 67c is an operation button for displaying the analysis results of the counseling information stored in the counseling information storage part 230. In the present embodiment, when the operation button 67c is operated, a screen 60 changes to a screen displaying the analysis results of the counseling information stored in the counseling information storage part 230.

The operation button 67d is an operation button for recording occurrence of an event during counseling, and may be displayed only during counseling. The event during counseling is an event found by the counselor. Specifically, the event during counseling may be, for example, a situation where the appearance of the counseling subject greatly changes during counseling.

In the present embodiment, when the operation button 67d is operated by the counselor during counseling, the information processing device 200 may display information indicating occurrence of an event in the display field 60a, with the timing at which the operation is received being regarded as the timing at which the event occurs. The information indicating occurrence of an event may be, for example, a marker or the like displayed on the waveform 61.

In the present embodiment, when the operation button 67d is operated by the counselor during counseling, the date and time at which the operation is received are included in a part of the counseling information as the timing at which the event occurs.

In the present embodiment, the operation button 67d is operated when the event occurs. This is by no means a limitation. Recording of the occurrence of the event may be performed, for example, by operating a communication terminal that is communicable with the information processing device 200.

In this case, when the counselor determines that the event occurs, the counselor can provide the information processing device 200 with a notification indicating the occurrence of the event by operating the transmitter near the counselor, and can record the timing of the occurrence of the event in the information processing device 200 without paying attention to the information processing device 200.

The display field 68a displays information indicating the time at which the obtainment of the biological information is started. In other words, the display field 68a displays information indicating the time at which the counseling is started. The display field 68b displays the elapsed time from the start of the counseling. The display field 68c stores information indicating the time at which the obtainment of the biological information is ended. In other words, the display field 68c displays the end time of the counseling. The display field 68d displays information identifying the counseling subject who is receiving the counseling.

In the present embodiment, for example, the information displayed in the display field 68a and the information displayed in the display field 68d may be used as identification information for identifying single-session counseling.

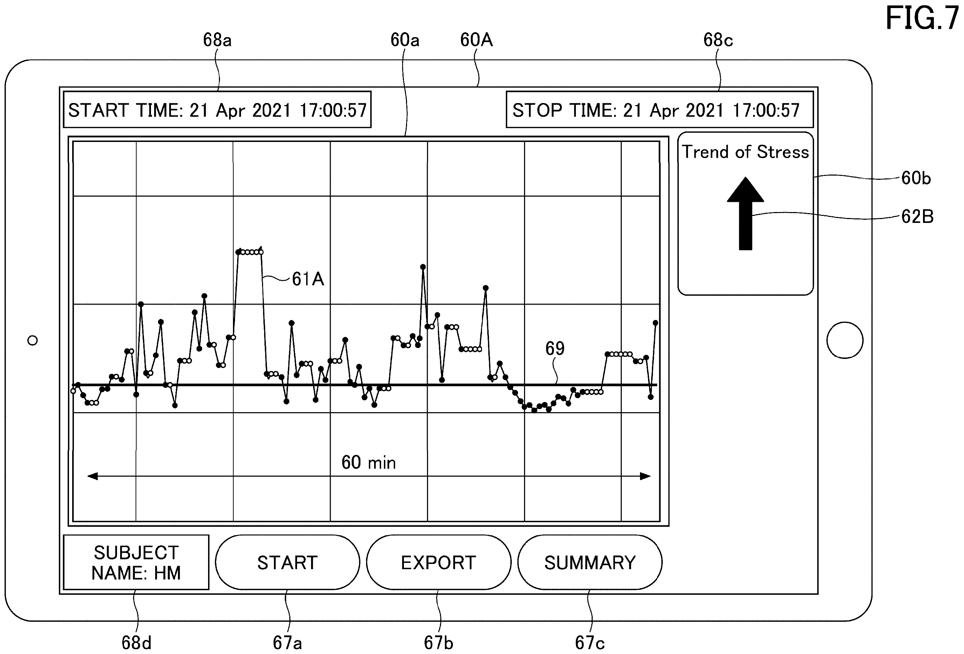

FIG. 7 is a second diagram illustrating an example of display of the information processing device. The screen 60A as illustrated in FIG. 7 illustrates a state after the end of the counseling.

In the screen 60A, the waveform 61A displayed in the display field 60a is a waveform until the end of the counseling.

The display field 60b displays an image 62A indicating the trend of the fluctuation in the stress value at the end of the counseling. In the example of FIG. 7, it can be found that the stress value at the end of the counseling is in an increasing trend.

The screen 60A displays a straight line 69 indicating the average value of the stress values in the counseling that is currently conducted. Further, in the present embodiment, information indicating the counseling period may be displayed. In the example of FIG. 7, "60 min" is displayed as the counseling period.

In the screen 60A, the operation button 67a is displayed as an operation button for starting a new counseling session. Further, in the screen 60A, the operation button 67d is hidden.

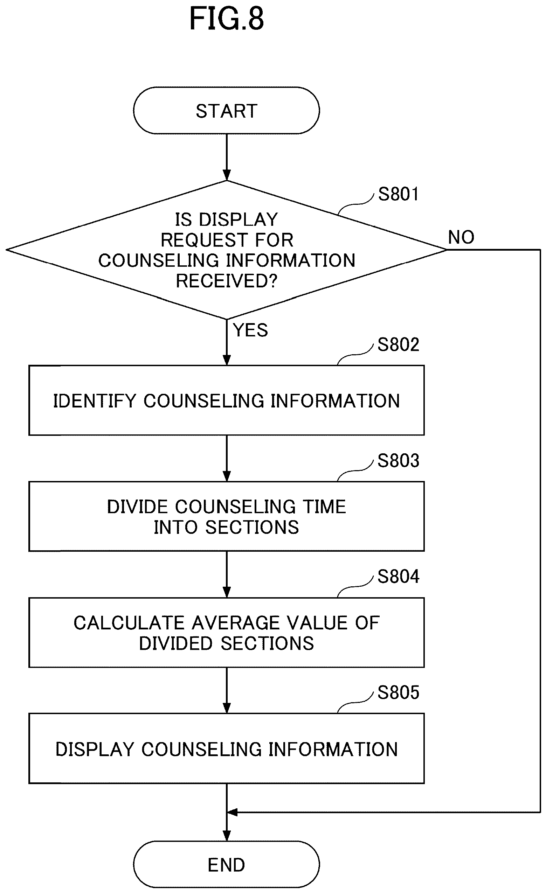

Next, the process performed by the information processing device 200 when the operation button 67c is operated in the screen 60A will be described with reference to FIG. 8. In the following description, it is assumed that before the operation button 67c is operated in the screen 60A, the operation button 67b is operated and counseling information is stored in the counseling information storage part 230.

FIG. 8 is a second flowchart describing a process performed by the information processing device. The input reception part 211 of the information processing device 200 of the present embodiment determines whether or not a display request for the counseling information is received (step S801). Specifically, the input reception part 211 determines whether or not the operation button 67c is operated in the screen 60A.

When the display request is not received in step S801, the information processing device 200 ends the process.

When the display request is received in step S801, the information storage part 217 of the information processing device 200 identifies the counseling information for which the display request is received, from the counseling information stored in the counseling information storage part 230 (step S802) .

In the present embodiment, the identification information displayed on the screen 60A may be included in the display request.

Subsequently, the information analysis part 218 of the information processing device 200 analyzes the waveform indicating the stress value included in the counseling information, and divides the counseling period into a plurality of sections (step S803) .

The counseling period is the time from the start until the end of counseling. In other words, the counseling period is a predetermined time range.

For example, the information analysis part 218 of the present embodiment may divide the counseling period by the trend of the fluctuation in the stress value, or may simply divide the counseling period into a predetermined number at equal intervals. Specifically, the information analysis part 218 may estimate a scenario in the counseling, for example, based on the fluctuation in the stress value, and divide the counseling period by the scenarios. The scenario in the counseling may be, for example, a scenario of building a relationship, questioning, or mindfulness.

In the present embodiment, the information analysis part 218 divides the counseling period into the plurality of sections. However, this is by no means a limitation. In the present embodiment, the counselor may divide the counseling period.

Subsequently, the information analysis part 218 calculates the average value of the stress values in the divided sections (step S804). Subsequently, the information processing device 200 displays counseling information reflecting the analysis results (step S805) and ends the process.

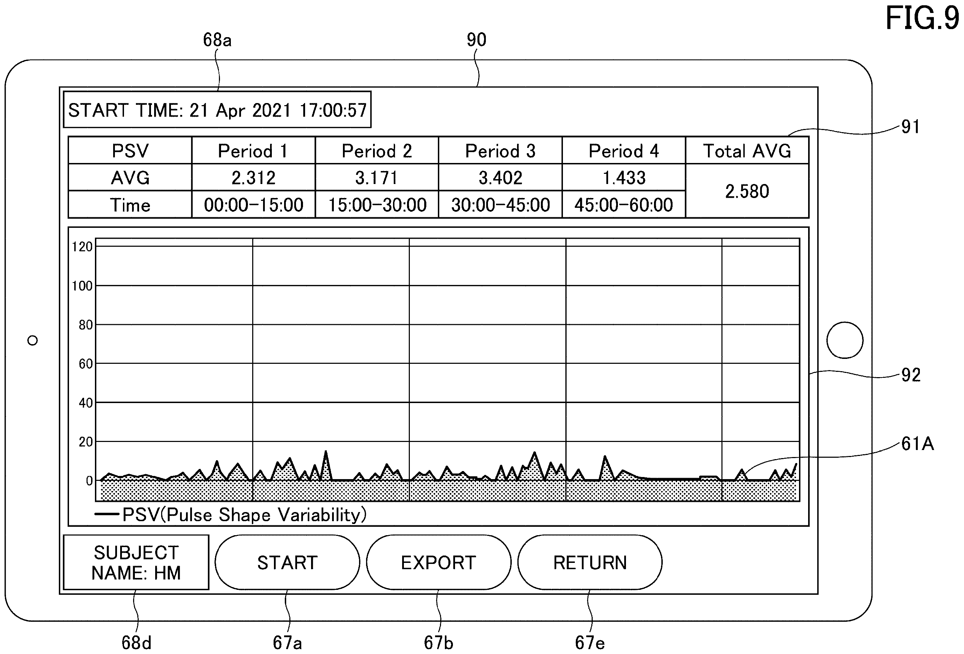

FIG. 9 is a third diagram illustrating an example of display of the information processing device. A screen 90 as illustrated in FIG. 9 is an example of a screen displayed on the information processing device 200 in step S805 of FIG. 8.

The screen 90 as illustrated in FIG. 9 includes display fields 91 and 92, operation buttons 67a, 67b, and 67e, and display fields 68a and 68d.

In the display field 91, the start time and the end time of a plurality of sections into which the counseling period is divided, are displayed in association with the average values of the stress values in the respective sections. In the example of FIG. 9, the counseling period is divided into four sections in which one section is set to 15 minutes. The waveform 61A indicating the stress value is displayed in the display field 92.

The operation button 67e is an operation button for changing the screen 90 to the screen 60A. In the present embodiment, when the operation button 67e is operated, the screen 90 changes to the screen 60A.

In the present embodiment, besides the display field 91, the average value of the heart rates or the like during counseling may be displayed for each of the sections. In other words, the biological information included in the counseling information may be displayed on the screen 90.

In the present embodiment, comments or the like may be input for each of the sections displayed in the display field 91. Specifically, in the information processing device 200 of the present embodiment, when "Period 1 (Section 1)" displayed in the display field 91 is selected, a comment input field may be popped up on the screen 90. When the counselor inputs comments to the comment input field and performs an operation for instructing to store the comments, the information processing device 200 may associate Period 1 with the input comments (text data) and store the associated comments in the counseling information storage part 230 as a part of the counseling information.

In the present embodiment, when the counseling information including comments is displayed on the screen 90, a display mode of the section associated with the comments may be made different from a display mode of the section not associated with the comments.

The following is an example assuming that when displaying the past counseling information stored in the counseling information storage part 230, the counseling information to be displayed includes comments associated with Section 1.

Specifically, the information processing device 200 differs the display mode of "Period 1" from the display modes of "Period 2", "Period 3", and "Period 4" with which comments are not associated. For example, "Period 1" may be highlighted.

In this case, when "Period 1" is selected, the information processing device 200 may pop up the comments associated with "Period 1".

In the present embodiment, by storing the comments in association with the respective sections into which the counseling period is divided, the counselor can refer to the record and consideration in relation to the counseling conducted in the past, in association with change in the psychological state of the counseling subject.

Therefore, according to the present embodiment, it is possible to assist the counselor to review the past counseling and plan the next counseling procedure.

As described above, in the present embodiment, information indicating the trend of the psychological state of the counseling subject during counseling is displayed on the information processing device 200 on the basis of the biological information of the counseling subject during counseling, thereby assisting the counselor to estimate the psychological state of the counseling subject.

Specifically, in the present embodiment, a waveform indicating the stress value of the counseling subject during counseling in real time in time series is displayed. In addition, in the present embodiment, an image indicating the trend of the fluctuation in the stress value is displayed together with the waveform.

Therefore, in the present embodiment, the counselor can confirm a reaction of the counseling subject during counseling on the basis of the stress value, and receive assistance in selecting a counseling technique in accordance with the reaction of the counseling subject.

In the present embodiment, the counselor receives assistance in this manner and can increase skills, leading to an increase in the quality of the counseling. As a result, withdrawal of the counseling subject from the counseling can be suppressed.

### (Second Embodiment)

A second embodiment will be described below with reference to the drawings. The second embodiment is different from the first embodiment in that counseling is conducted via a network, such as the Internet or the like. In the following description of the second embodiment, the difference from the first embodiment will be described, and the same functional configurations as in the first embodiment are denoted by the same symbols as in the first embodiment and description thereof will be omitted.

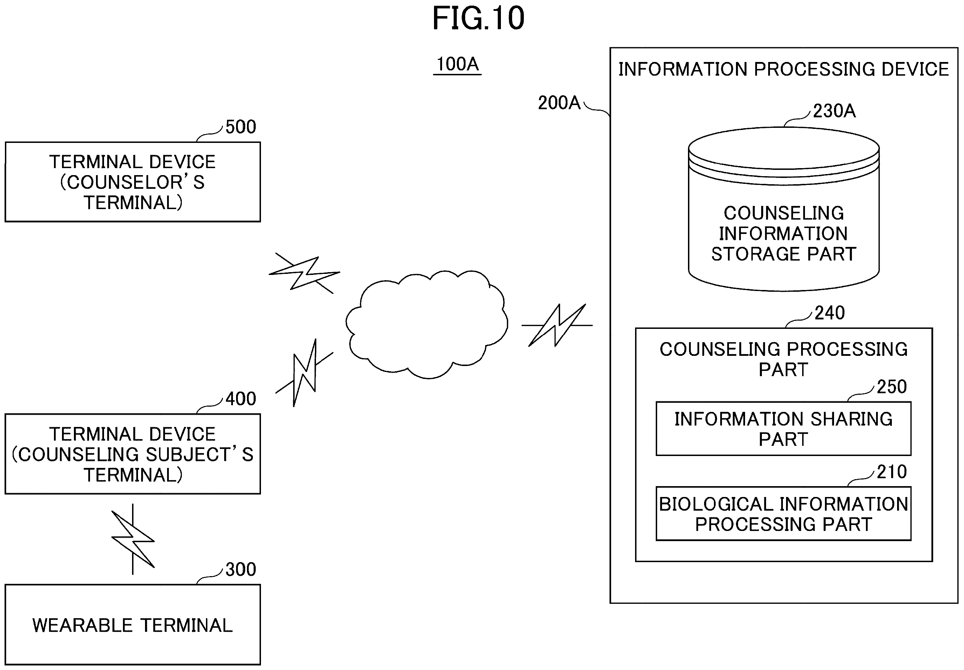

FIG. 10 is a diagram illustrating an example of a system configuration of a biological information processing system of a second embodiment.

A biological information processing system 100A of the present embodiment includes an information processing device 200A, a wearable terminal 300, a terminal device 400, and a terminal device 500. In the biological information processing system 100A, the information processing device 200A, the terminal device 400, and the terminal device 500 are connected via a network, such as the Internet or the like. The wearable terminal 300 is connected to the terminal device 300 via a near field wireless communication or the like.

The terminal device 400 of the present embodiment is mainly used by a counseling subject wearing the wearable terminal 300. In the following description, the terminal device 400 will be referred to as a counseling subject's terminal 400. The counseling subject's terminal 400 performs wireless communication with the wearable terminal 300 to obtain biological information 10 of the counseling subject during counseling detected by the wearable terminal 300. The counseling subject's terminal 400 transmits the biological information 10 obtained from the wearable terminal 300 to the information processing device 200A.

The terminal device 400 may also include a capturing device and transmits image data captured by the capturing device to the information processing device 200A. The image data transmitted to the information processing device 200A may be image data obtained by capturing the counseling subject.

The terminal device 500 of the present embodiment is mainly used by a counselor. In the following description, the terminal device 500 will be referred to as a counselor's terminal 500. The terminal device 500 may include a capturing device and transmits image data captured by the capturing device to the information processing device 200A. The image data transmitted to the information processing device 200A may be image data obtained by capturing the counselor. The image data of the present embodiment includes still image data and moving image data.

The counseling subject's terminal 400 and the counselor's terminal 500 of the present embodiment may be a tablet-type terminal device, a smartphone, or the like. The counseling subject's terminal 400 and the counselor's terminal 500 may be a desktop computer or the like.

The information processing device 200A of the present embodiment is, for example, a server device installed on the Internet. The information processing device 200A includes a counseling information storage part 230A and a counseling processing part 240.

The counseling information storage part 230A is configured to store counseling information in the present embodiment. Specifically, the counseling information of the present embodiment includes biological information obtained from the counseling subject's terminal 400, stress values, a trend of fluctuation in the stress value, analysis results of stress values, text data input by the counselor, and image data of the counseling subject obtained by capturing the counseling subject during counseling.

The counseling information may also include identification information for identifying the counseling subject and information for identifying the date and time at which the counseling is conducted.

A counseling processing part 240 realizes counseling via the Internet communication.

The counseling processing part 240 of the present embodiment includes an information sharing part 250 and a biological information processing part 210. The information sharing part 250 causes the terminal device 400 and the terminal device 500 to share a screen.

Specifically, when counseling is started, the information sharing part 250 causes a counseling subject's screen to be displayed on the counseling subject's terminal 400 and causes a counselor's screen to be displayed on the counselor's terminal 500. Then, the information sharing part 250 causes the image data transmitted from the counseling subject's terminal 400 and the counselor's terminal 500 to be displayed on the counseling subject's screen and the counselor's screen.

In the present embodiment, by causing each other's images to be displayed on the counseling subject's terminal 400 and the counselor's terminal 500 in this manner, it is possible to realize counseling in a state in which the counseling subject and the counselor are in physically remote areas.

The counseling processing part 240 of the present embodiment may be realized by a dedicated application for conducting counseling. In this case, the biological information processing part 210 and the information sharing part 250 are functions realized by the dedicated application.

The counseling processing part 240 may be, for example, realized by adding the functions of the biological information processing part 210 to an existing communication tool configured to realize the information sharing part 250. The existing communication tool may have, for example, an information sharing function and realize online meetings and the like.

In the example of FIG. 10, the number of the information processing devices 200A included in the biological information processing system 100A is one. However, this is by no means a limitation. The information processing device 200A may be realized by a plurality of information processing devices. In the example of FIG. 10, the counseling information storage part 230A is provided in the information processing device 200A. However, this is by no means a limitation. The counseling information storage part 230A may be partially or entirely provided in an external device configured to communicate with the information processing device 200A.

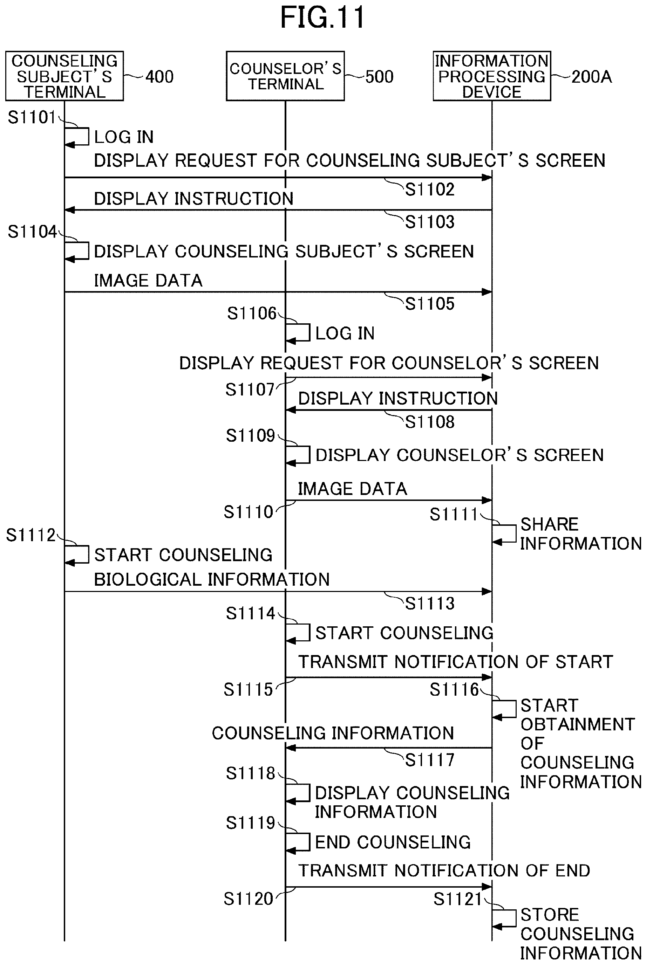

Next, a process performed by the biological information processing system 100A of the present embodiment will be described with reference to FIG. 11. FIG. 11 is a sequence diagram illustrating a process performed by the biological information processing system of the second embodiment.

In the biological information processing system 100A of the present embodiment, when the counseling subject performs a login operation to the biological information processing system 100A (step S1101), the counseling subject's terminal 400 transmits a display request for the counseling subject's screen to the information processing device 200A (step S1102).

The counseling processing part 240 of the information processing device 200A receives the display request and transmits a display instruction of the counseling subject's screen to the counseling subject's terminal 400 (step S1103).

The counseling subject's terminal 400 receives the display instruction and displays the counseling subject's screen (step S1104). Subsequently, when the counseling subject's screen is displayed, the counseling subject's terminal 400 starts transmitting the image data of the counseling subject captured by the capturing device included in the counseling subject's terminal 400 to the information processing device 200A (step S1105).

In the biological information processing system 100A, when the counselor performs a login operation to the biological information processing system 100A (step S1106), the counselor's terminal 500 transmits a display request for the counselor's screen to the information processing device 200A (step S1107).

The counseling processing part 240 of the information processing device 200A receives the display request and transmits a display instruction of the counselor's screen to the counselor's terminal 500 (step S1108).

The counselor's terminal 500 receives the display instruction and displays the counselor's screen (step S1109). Subsequently, when the counselor's screen is displayed, the counselor's terminal 500 starts transmitting the image data of the counselor obtained by capturing the counselor by the capturing device included in the counselor's terminal 500 to the information processing device 200A (step S1110).

When the information processing device 200A receives the image data from the counseling subject's terminal 400 and the counselor's terminal 500, the information sharing part 250 causes the counseling subject's terminal 400 and the counselor's terminal 500 to share the image data (step S1111).

Specifically, for example, the information sharing part 250 displays the image data of the counselor on the counseling subject's terminal 400 and displays the image data of the counseling subject on the counselor's terminal 500.

Subsequently, when the counseling subject's terminal 400 receives a counseling subject's operation of an instruction to start counseling (step S1112), the counseling subject's terminal 400 starts transmitting biological information received from the wearable terminal 300 to the information processing device 200A (step S1113) .

When the counselor's terminal 500 receives an operation of a counselor's instruction to start counseling (step S1114), the counselor's terminal 500 transmits a notification of the start of counseling to the information processing device 200 (step S1115) .

The biological information processing part 210 of the information processing device 200A receives the notification of the start of counseling and starts obtainment of counseling information (step S1116). In other words, the biological information processing part 210 starts storing the counseling information.

Subsequently, the information processing device 200A transmits the obtained counseling information to the counselor's terminal 500 (step S1117). The counselor's terminal 500 displays the received counseling information on the counselor's screen (step S1118).

Subsequently, when the counselor's terminal 500 receives a counselor's operation of an instruction to end the counseling (step S1119), the counselor's terminal 500 transmits a notification of the end of counseling to the information processing device 200 (step S1120) .

The biological information processing part 210 of the information processing device 200A receives the notification of the end of counseling and stores the counseling information in the counseling information storage 230A (step S1121).

The process of from step S1116 to step S1118 in FIG. 11 is the same as the process of from step S502 to step S510 in FIG. 5 except that the display destination of display of the waveform indicating the fluctuation in the stress value and the information indicating the trend of the fluctuation is the counselor's terminal 500.

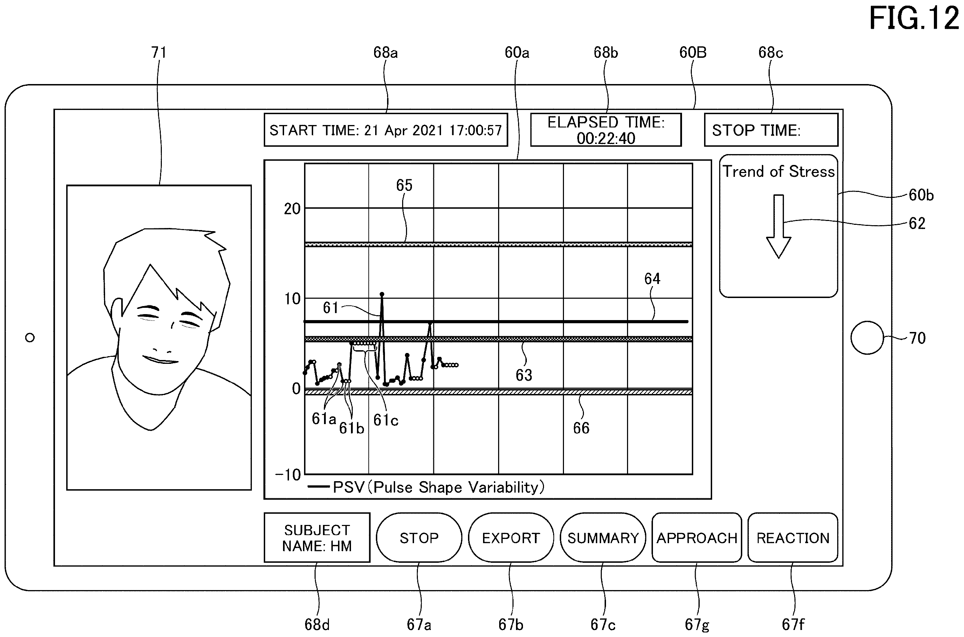

Next, the counselor's screen displayed on the counselor's terminal 500 of the present embodiment will be described with reference to FIG. 12.

FIG. 12 is a diagram illustrating an example of the counselor's screen of the second embodiment. The counselor's screen is a screen displayed on the counselor's terminal 500 during counseling. A screen 60B as illustrated in FIG. 12 is, for example, displayed on the counselor's terminal 500 in step S1118 of FIG. 11.

The screen 60B includes display fields 60a and 60b, operation buttons 67a, 67b, 67c, 67f, and 67g, and display fields 68a, 68b, 68c, 68d, and 71. The information processing device 200 includes an operation button 70 (input device).

The display field 71 of the present embodiment displays a face image or the like of the counseling subject captured by the counseling subject's terminal 400.

The operation button 67g is selected, for example, when the counselor conducts a dialogue related to the core of a counseling subject's issue. In other words, the operation button 67g is selected by the counselor when the counselor conducts a dialogue that can change the psychological state of the counseling subject.

The operation button 67f is selected by the counselor when some change appears in the psychological state of the counseling subject. Specifically, the operation button 67f may be selected, for example, when a large fluctuation is observed in the amplitude of the waveform 61 indicating the fluctuation in the stress value displayed in the display field 60a, or when the trend of the fluctuation in the stress value is changed in the display field 60b. In addition, the operation button 67f may be selected when the counselor determines that the appearance of the counseling subject changes based on the image of the counseling subject displayed in the display field 71.

In the present embodiment, in the counseling information, the timing at which the operation button 67g and the operation button 67f are selected may be stored in association with the biological information.

Thus, in the present embodiment, by storing the timing of selection of the operation button 67g in association with the biological information, it is possible to record the timing at which the counselor starts a dialogue that might influence the psychological state of the counseling subject.

Further, in the present embodiment, by storing the timing of selection of the operation button 67f in association with the biological information, it is possible to record a reaction of the counseling subject, for example, when a dialogue that might influence the psychological state of the counseling subject is started.

In the present embodiment, for example, the information processing device 200A may include a facial expression recognition function using image data. In this case, the information processing device 200A may recognize the facial expression of the counseling subject using image data transmitted from the counseling subject's terminal 400, and display the result in the display field 60a.

Specifically, for example, the information processing device 200A may recognize the facial expression of the counseling subject at predetermined intervals, and display an icon image indicating the result of the facial expression recognition together with the waveform 61 in the display field 60a at each of the timings at which the facial expression recognition is conducted. In the present embodiment, information associating the result of the facial expression recognition with the timing of the facial expression recognition may be stored in the counseling information storage part 230 as a part of the counseling information.

In the present embodiment, by displaying the result of the facial expression recognition using the face image of the counseling subject together with the waveform 61, the counselor can receive assistance in understanding the psychological state of the counseling subject.

In addition, the information processing device 200A may comprehensively determine the psychological state of the counseling subject on the basis of audio data and biological information of the counseling subject in combination with the facial expression recognition based on the image data of the counseling subject. The information processing device 200A may display the determination result of the psychological state together with the waveform 61 in the display field 60a at each of the timings at which the determination is performed. The psychological state of the counseling subject includes, for example, a nervous state, a relaxed state, and the like.

In the present embodiment, by displaying the determination result of the psychological state of the counseling subject together with the waveform 61, the counselor can receive assistance in understanding the psychological state of the counseling subject.

The present invention should not be construed as being limited to the configurations described above, and it is possible to combine the configurations described in the above embodiments with other elements. These can be changed to the extent that they do not deviate from the gist of the present invention, and can be appropriately defined in accordance with an intended form of application.

Also, the present international application claims priority to Japanese Patent Application No. 2021-157513, filed on September 28, 2021 and Japanese Patent Application No. 2022-065511, filed on April 12, 2022, and the entire contents of Japanese Patent Application No. 2021-157513 and Japanese Patent Application No. 2022-065511 are incorporated in this international application by reference.

### REFERENCE SIGNS LIST

100, 100a biological information processing system
200, 200a information processing device
210 biological information processing part
211 input reception part
212 biological information obtainment part
213 information determination part
214 stress value calculation part
215 trend determination part
216 display control part
217 information storage part
218 information analysis part
230 counseling information storage part
240 counseling processing part
250 information sharing part
300 wearable terminal
400, 500 terminal device

## Claims

1. A biological information processing program that causes a computer to execute a process comprising:
obtaining biological information of a user from a biological information obtainment device; and
causing a display device to display both a waveform indicating time-series change in the biological information and information indicating a result of processing using a predetermined number of pieces of the biological information.

2. The biological information processing program according to claim 1, wherein
the information indicating the result of the processing is information indicating a trend of change in the biological information.

3. The biological information processing program according to claim 1 or 2, wherein
the information indicating the result of the processing is an image indicating an increase or decrease in a value calculated based on the biological information.

4. The biological information processing program according to any one of claims 1 to 3, wherein
the biological information processing program causes the computer to execute a process of performing switching in accordance with an operation between a displayed state and a hidden state of
the waveform indicating the time-series change in the biological information, or
the information indicating the result of the processing using the biological information.

5. The biological information processing program according to any one of claims 1 to 4, wherein
the biological information processing program causes the computer to execute a process of
calculating an index value indicating a magnitude of a psychological stress of the user, using the biological information, and
displaying a waveform indicating change in the index value as the waveform indicating the time-series change in the biological information.

6. The biological information processing program according to claim 5, wherein
the biological information processing program causes the computer to execute a process of
determining whether or not the obtained biological information is valid biological information, and
calculating the index value using the biological information determined to be valid.

7. The biological information processing program according to claim 5 or 6, wherein
the information indicating the result of the processing is information indicating an average value of the index value.

8. The biological information processing program according to any one of claims 5 to 7, wherein
the biological information is biological information obtained for a counseling period during which the user is receiving counseling.

9. The biological information processing program according to claim 8, wherein
counseling information is stored in a storage part after an end of the counseling, the counseling information including the waveform indicating time-series change in the index value and the information indicating the result of the processing using the predetermined number of the pieces of the biological information, and
in response to receiving a display request for the counseling information, the display device displays the waveform indicating the time-series change in the index value and the information indicating the result of the processing using the predetermined number of the pieces of the biological information included in the counseling information.

10. The biological information processing program according to claim 9, wherein
the display device further displays an average value of the index value calculated from biological information obtained for a period of a most-recent prior counseling of the user.

11. The biological information processing program according to claim 9 or 10, wherein
the biological information processing program causes the computer to execute a process of
dividing the counseling period into a plurality of sections, and
causing the display device to display the information indicating the result of the processing using the biological information obtained in each of the plurality of sections together with the waveform indicating the time-series change in the index value for each of the plurality of sections.

12. The biological information processing program according to claim 11, wherein
the plurality of sections are sections obtained by dividing the counseling period by scenarios in the counseling.

13. The biological information processing program according to claim 11 or 12, wherein
an input of text data in association with the sections is received, and
information associating the text data with the sections is included in the counseling information.

14. The biological information processing program according to any one of claims 1 to 13, wherein
the biological information processing program causes the computer to execute a process of
in response to receiving an operation indicating occurrence of an event during obtainment of the biological information, storing information indicating that the event occurs in a storage part, and
displaying the information indicating that the event occurs on the waveform indicating the time-series change in the biological information.

15. The biological information processing program according to any one of claims 1 to 14, wherein
the biological information processing program causes the computer to execute a process of
causing the display device to display image data obtained by capturing the user together with both the waveform indicating the time-series change in the biological information and the information indicating the result of the processing using the predetermined number of the pieces of the biological information.

16. The biological information processing program according to claim 15, wherein
facial expression recognition of the user is performed based on the image data, and
the display device displays a result of the facial expression recognition together with both the waveform indicating the time-series change in the biological information and the information indicating the result of the processing using the predetermined number of the pieces of the biological information.

17. An information processing device, comprising:
a biological information obtainment part configured to obtain biological information of a user from a biological information obtainment device; and
a display control part configured to cause a display device to display both a waveform indicating time-series change in the biological information and information indicating a result of processing using a predetermined number of pieces of the biological information.

18. A biological information processing system, comprising:
a biological information obtainment device; and
an information processing device configured to communicate with the biological information obtainment device, wherein
the information processing device includes
a biological information obtainment part configured to obtain biological information of a user from the biological information obtainment device, and
a display control part configured to cause a display device to display both a waveform indicating time-series change in the biological information and information indicating a result of processing using a predetermined number of pieces of the biological information.

19. A biological information processing method, which is performed by a biological information processing system including a biological information obtainment device and an information processing device configured to communicate with the biological information obtainment device, the biological information processing method comprising:
obtaining biological information of a user from the biological information obtainment device; and
causing a display device to display both a waveform indicating time-series change in the biological information and information indicating a result of processing using a predetermined number of pieces of the biological information,
the obtaining and the displaying being performed by the information processing device.
